(19) Europäisches Patentamt
European Patent Office
Office européen des brevets

(11) **EP 1 965 193 B1**

(12) **EUROPÄISCHE PATENTSCHRIFT**

(45) Veröffentlichungstag und Bekanntmachung des Hinweises auf die Patenterteilung:
**29.04.2015 Patentblatt 2015/18**

(21) Anmeldenummer: **08450020.6**

(22) Anmeldetag: **25.02.2008**

(51) Int Cl.:
*G01N 21/05* (2006.01)   *G01N 21/31* (2006.01)
*G01N 21/35* (2014.01)   *G01N 33/14* (2006.01)
*G01J 3/02* (2006.01)   *G01J 3/10* (2006.01)
*G01J 3/42* (2006.01)

(54) **Verfahren und Vorrichtung zur Ermittlung des Alkoholgehaltes von Flüssigkeiten**

Method and apparatus for determining the alcohol content of fluids

Procédé et dispositif de détermination de la teneur en alcool de liquides

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **01.03.2007 AT 3302007**

(43) Veröffentlichungstag der Anmeldung:
**03.09.2008 Patentblatt 2008/36**

(73) Patentinhaber: **Anton Paar GmbH**
**8054 Graz-Straßgang (AT)**

(72) Erfinder: **Benes, Roman**
**8055 Graz (AT)**

(74) Vertreter: **Wildhack & Jellinek**
**Patentanwälte**
**Landstraßer Hauptstraße 50**
**1030 Wien (AT)**

(56) Entgegenhaltungen:
WO-A-00/50893   WO-A-03/065021
DE-A1- 3 407 844   GB-A- 2 288 231
US-A- 4 553 033   US-A- 5 679 955

- **"Alocolyzer Wein - Alkoholmessgerät für Wein"[Online] April 2005 (2005-04), Seiten 1-6, XP002489025 Gefunden im Internet: URL:http://www.anton-paar.com/ap/apinterne t/html/default/cxsn-5r9kh2.en.0.jsp> [gefunden am 2008-07-10]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung bezieht sich auf ein neues Verfahren zur spektroskopischen Bestimmung der Konzentration von Alkoholen, insbesondere von einwertigen Alkoholen, vorzugsweise von Ethanol, in flüssigen Proben, insbesondere in alkohol- bzw. ethanolhaltigen Nahrungsmitteln, Heilmitteln, kosmetischen Produkten und dgl. und auf eine Vorrichtung zur Durchführung des Verfahrens.

Die spektroskopische Bestimmung der Konzentration von Alkoholen, insbesondere Ethanol, in flüssigen Proben ist eine weit verbreitete Methode, wobei es mittels Nah-Infrarot-Spektroskopie (NIR) gelingt, verschiedenste Parameter qualitativ und/oder quantitativ zu bestimmen.

Der Wellenlängenbereich in der NIR-Spektroskopie erstreckt sich hierbei von etwa 700 bis etwa 2500 nm, wobei jedoch bei diesem Verfahren üblicherweise nicht Grundschwingungen, sondern Ober- und Kombinationsschwingungen gemessen werden. In diesem Wellenlängenbereich sind jedoch die Absorptionsfähigkeiten der zu untersuchenden Substanzen geringer und die Absorptionsbanden breiter und überlappen bzw. überlagern einander häufig, was eine Interpretation der Messergebnisse schwierig und manchmal eindeutige Messungen unmöglich macht.

Durch die Herstellung und Verfügbarkeit billiger Bauteile, welche für spektroskopische Untersuchungen in diesem Wellenbereich eingesetzt werden können, wie beispielsweise auf dem Gebiet der Glasoptik, der Halbleiterdetektoren und neuer Lichtquellen, sind jedoch die Möglichkeiten einer einfachen und raschen Untersuchung von Proben mittels NIR-Spektroskopie unter Einsatz von relativ einfachen Vorrichtungen zur Durchführung derartiger Verfahren wesentlich breiter geworden.

Ein Verfahren der eingangs genannten Art zur spektroskopischen Bestimmung der Konzentration von niedrigen Alkoholen, insbesondere Ethanol, in flüssigen Proben ist beispielsweise aus der US 5,679,955 A ("US-A") bekannt geworden, wobei bei jeweils einer einzigen Wellenlänge im nahen Infrarotbereich Transmissionsmessungen vorgenommen werden. Der Wellenlängenbereich zur Durchführung der Messungen unter Berücksichtigung der zum weitaus überwiegenden Teil in einer alkoholhaltigen Probe enthaltenen Bestandteile Ethanol und Wasser ist bei diesem bekannten Verfahren derart gewählt, dass die Absorption durch das in der Probe enthaltene Wasser gegenüber der Absorption durch den darin zusätzlich enthaltenen Alkohol überwiegt.

Es werden eine Vielzahl von Kalibriermessungen bei jeweils einer Wellenlänge durchgeführt, worauf der Alkoholgehalt von weiteren Proben auf Basis der erhaltenen Kalibrierdaten bestimmt wird, wobei gemäß diesem bekannten Stand der Technik vorgeschlagen wird, unter Berücksichtigung der Absorptionskoeffizienten von Wasser und Ethanol die Messungen bei Wellenlängen von etwa 0,98 $\mu$m, etwa 1,3 $\mu$m und etwa 1,45 $\mu$m durchzuführen.

Nachteilig bei diesem bekannten Verfahren ist die Tatsache, dass die Messungen somit bei Wellenlängen durchgeführt werden, bei welchen der Absorptionskoeffizient von Wasser jenen von Ethanol überwiegt, sodass eine Bestimmung des Alkoholgehaltes nur mit einer entsprechend hohen Ungenauigkeit vorgenommen werden kann und weiters die Bestimmung des Alkoholgehaltes gemäß dieser US-A auf Alkoholgehalte von weniger als 10 Vol.-% Alkohol beschränkt ist.

Es ist somit dieses bekannte Verfahren im wesentlichen auf die Bestimmung der Alkoholkonzentration von Bieren beschränkt, während Getränke bzw. allgemein flüssige Proben mit einem höheren Alkoholgehalt, wie beispielsweise Weine, Brände, Heilmittel oder auch kosmetische Produkte, mit diesem bekannten Verfahren nicht untersucht werden können. Darüber hinaus ist eine Berücksichtigung von in den zu untersuchenden Proben enthaltenden Drittsubstanzen, welche das Messergebnis teilweise beträchtlich beeinflussen können, bei Anwendung dieses bekannten Verfahrens nicht ohne weiteres möglich.

Die Hardware für diese Methode ist bevorzugt mittels einem oder mehreren Interferenzfilter(n) realisiert.

Eine weitere Methode nach WO 00/50893 A1 bzw. ("US-B1") US 6,690,015 B1 nutzt zur Alkoholbestimmung die Absorption von IR-Licht durch die zu untersuchende Probe bei wenigstens einer Wellenlänge im Bereich von 1100 nm bis 1300 nm, bevorzugt im Bereich von 1150 nm bis 1250 nm. In diesem Spektralbereich weisen Alkohole ein stark ausgeprägtes Absorptionsmaximum auf und sind somit leicht von dem Absorptionsspektrum des zum überwiegenden Teil in derartigen Proben befindlichen Wassers zu unterscheiden.

Bei einer Messung der Absorption in diesem Wellenlängenbereich lässt sich nach einer Kalibration, welche vorab unter Verwendung entweder einer künstlichen oder realen Probe, beispielsweise ein Ethanol-Wasser-Gemisch, Bier, Wein od. dgl. erfolgte, unmittelbar der Alkoholgehalt der zu untersuchenden Probe feststellen.

Neben Alkohol und Wasser sind jedoch teilweise Drittsubstanzen enthalten, welche die Bestimmung der Ethanol-Konzentration in der zu untersuchenden Probe beeinflussen können. Die US-B1 beschreibt Auswahlkriterien von Arbeitswellenlängen aus dem Wellenlängenbereich, wo das Absorptionsverfahren von in der Probe enthaltenden Zusatzstoffen ein im wesentlichen lineares Verhalten aufweist, wodurch es in einfacher Weise gelingt, die durch derartige Zusatzstoffe hervorgerufenen Einflüsse bei der Messung der Absorption und bei der Bestimmung der Konzentration zu eliminieren. Auf diese Weise ist die Alkoholbestimmung weitgehend probenunabhängig in einem Konzentrationsbereich bis 60 % Alkohol hinreichend genau.

Die Methode gemäß dieser US-B1 verlangt hohe spektrale Auflösung, es muss daher ein Monochromator zum Einsatz kommen.

Die vorliegende Erfindung zielt nun darauf ab, ein spektroskopisches Verfahren zur Bestimmung des Alkohol-, insbesondere Ethanolgehaltes in wie oben genannten flüssigen Proben zur Verfügung zu stellen, mit welchem die Bestimmung des Alkohol-, insbesondere Ethanolgehaltes über einen weiten Konzentrationsbereich auf einfache Weise und extrem kostengünstig vorgenommen werden kann.

Zur Lösung dieser Aufgabe wird gemäß der Erfindung ein Verfahren gemäß Oberbegriff des Anspruches 1 vorgeschlagen, das die im Kennzeichen Teil dieses Anspruches angeführten Merkmale aufweist.

Es kommt also Absorption des IR-Lichts bei zumindest zwei unterschiedlichen Wellenlängen zum Einsatz. Diese Wellenlängen liegen im (+/-)-Nahbereich von bzw. bei 1200 nm, wo Wasser und Alkohol etwa gleich hoch absorbieren, und bei 1300 nm und/oder 1450 nm, wo die beiden Absorptionen sehr unterschiedlich sind.

Ein ganz wesentlicher Vorteil der Erfindung besteht darin, dass - über die bekannte Verwendung der Infrarot-Absorption im NIR-Bereich für die Charakterisierung des Alkoholgehaltes hinausgehend - mit billigen LEDs mit breitbandiger Strahlungscharakteristik und unter Verzicht auf teure Monochromatoren bei unterschiedlichen Wellenlängen mit einem an sich unspezifischen Detektor gemessen wird.

Durch gleichzeitige einfache Temperaturmessung und rechnerische Kompensation dieses Einflusses kann weiters auch ein aufwändiges Temperierbauteil entfallen. Dies ermöglicht eine neue Kategorie von Geräten, welche "handheld", also klein und handlich, einfach bedienbar und unproblematisch transportabel, kostengünstig und billig sind und welche einfach zu bedienen sind. Deren Kalibrierung ist ohne großen Aufwand und ohne nähere Kenntnisse von jedermann durchführbar.

Im Vergleich dazu wiegt ein handelsüblicher "Alcolyzer" zumindest etwa 10 kg und besitzt einen eingebauten Monochromator, ist also ungleich teurer.

Wesentlich für das erfindungsgemäße Verfahren ist die Nutzung des linearen Zusammenhangs zwischen der Alkoholkonzentration der Proben und der Absorption des IR-Lichts durch die Probe bei der jeweiligen Wellenlänge.

Der Anspruch 2 betrifft eine besondere Ausführungsform des neuen Verfahrens.

Gemäß Anspruch 3 ist der Einsatz einer IR-Strahlung mit zu den beiden erstgenannten unterschiedlichen Wellenlängen unterschiedlichen dritten Wellenlängen vorgesehen, wobei mit "einer Wellenlänge" im Rahmen der vorliegenden Erfindung immer ein kleiner Wellenlängenbereich gemeint ist, der beispielsweise etwa +/- 50nm um die genau angegebene Wellenlänge liegt.

Die Ansprüche 5 und 6 geben Auskunft über die für eine möglichst genaue Analyse vorteilhaften Stellen im Absorptions-Spektrum der NIR-Strahlung.

Dem Anspruch 7 können die für die Bestimmung des Gehaltes an Ethanol im Rahmen der Erfindung am günstigsten eingesetzten NIR-Wellenlängen entnommen werden.

Erfindungsgemäß ist das von der Lichtquelle abgestrahlte NIR-Licht keineswegs scharf monochromatisch, sondern der entsprechende Wellenlängenpeak ist breiterbandig, mit einer Halbwertsbreite von 50 bis 100 nm.

Derartige Halbwertsbreiten besitzen Halbleiterleuchtdioden, also LEDs. Gemäß der vorliegenden Erfindung werden bevorzugterweise IR-Lichtquellen mit 2 oder 3 LEDs eingesetzt, wobei jede der LEDs, die ihr eigene Wellenlänge abstrahlt, und die 2 oder 3 unterschiedlichen Wellenlängen gleichzeitig abgestrahlt werden, wobei man weder einen schmalen Interferenzfilter noch einen Monochromator benötigt. Hiebei wird jede der LEDs mit einer unterschiedlichen Frequenz moduliert und die vom Detektor aufgenommene Lichtenergie wird dann wieder demoduliert, so dass das die Probe durchdringende und dort unterschiedlich geschwächte IR-Licht jeder der LEDs gesondert messbar ist.

Gemäß einer anderen erfindungsgemäß einsetzbaren Methode wird bevorzugterweise zeitlich rasch nacheinander von jeder der LEDs das ihr eigene IR-Licht mit seiner individuellen Wellenlänge emittiert und der Detektor misst für jede Wellenlänge gesondert die Intensität des aus der Probe austretenden IR-Lichts. Selbstverständlich ist auch bei dieser Methode kein Interferenzfilter oder Monochromator nötig.

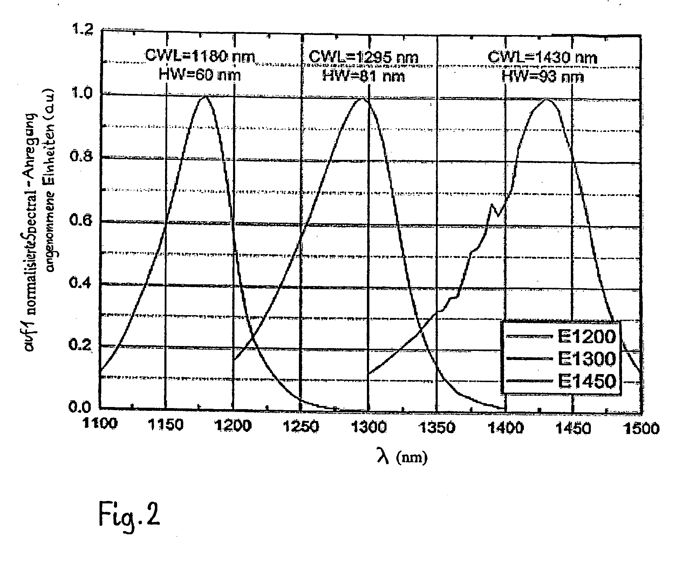

Das Emissionsspektrum von solchen LEDs ist in Fig. 2 gezeigt, wobei die Halbwertsbreite der Peaks hier etwa 70 - 90 nm beträgt (Anspruch 4).

Der Anspruch 8 betrifft eine die Genauigkeit der Alkoholbestimmung weiter verbessernde Korrektur von Intensitätsschwankungen der IR-Strahlung.

Aus dem Anspruch 9 gehen die Genauigkeit der Messung erhöhende Maßnahmen hervor.

Über eine günstige Form der Auswertung der Messergebnisse der IR-Detektoren gibt der Anspruch 10 Auskunft.

Gemäß dem Anspruch 11 können eine genaue Bestimmung der Temperatur der zu untersuchenden Flüssigkeit und/oder eine möglichst exakte Konstanthaltung dieser Temperatur vorgesehen sein.

Weiters betrifft der Anspruch 12 eine im Rahmen der Erfindung vorteilhafte Einrichtung zur Durchführung des neuen Alkoholgehalts-Bestimmungsverfahren gemäß den vorangegangenen Ansprüchen.

Schließlich betrifft der Anspruch 13 eine im Rahmen der Erfindung vorgesehene, die Genauigkeit der Messung erhöhende Einrichtung zur Kompensation von Schwankungen der Intensität der von der IR-Strahlungsquelle abgegebenen Strahlung.

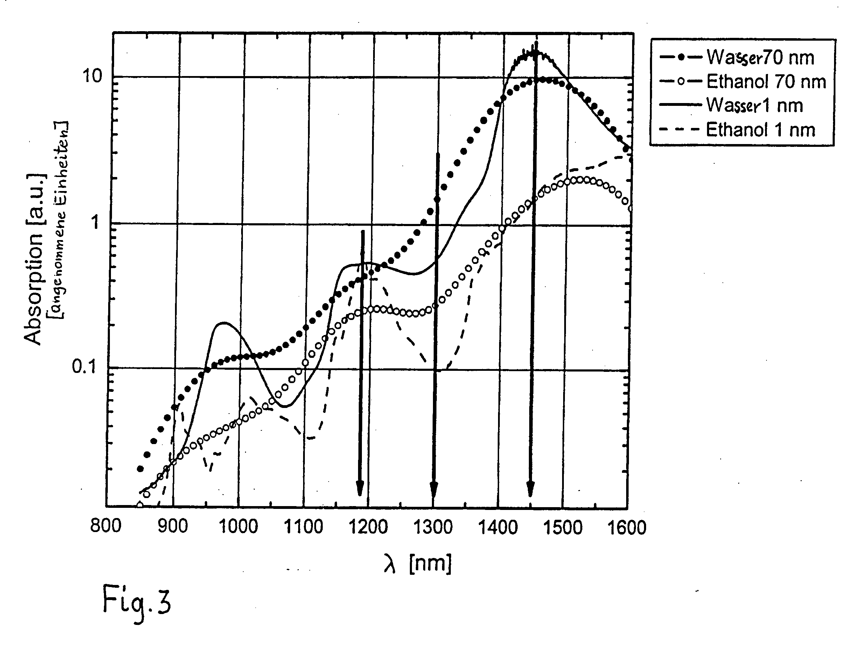

Der Einfluss der spektralen Auflösung auf die Absorptionsspektren von Wasser und Ethanol ist sehr gut aus der Fig. 3 ersichtlich.

Bei einer schlechteren spektralen Auflösung verschwinden zwar feine Strukturen aus dem Spektrum, dennoch unterscheiden sich die Spektren durchaus bedeutsam voneinander und können, wie gefunden wurde, somit ohne größere Probleme zur Konzentrationsbestimmung von Alkoholen, insbesondere Ethanol, herangezogen werden.

Neben dem Alkohol und Wasser sind in realen Proben sehr oft zumindest Drittsubstanzen enthalten, welche die Bestimmung der Ethanol-Konzentration in der zu untersuchenden Probe beeinflussen können.

Um den tatsächlichen Alkoholgehalt bestimmen zu können, müssen die Drittsubstanzen bei den Messwellenlängen unterschiedliche Beiträge zu der Absorption liefern.

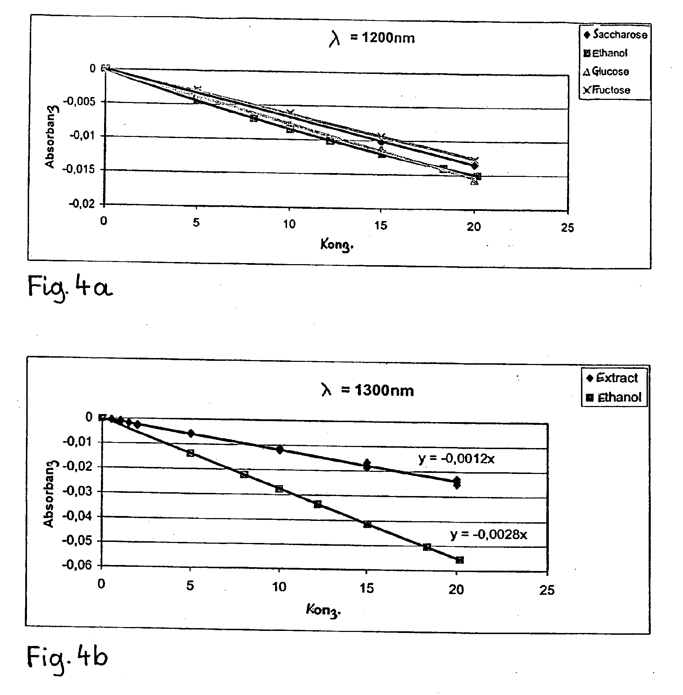

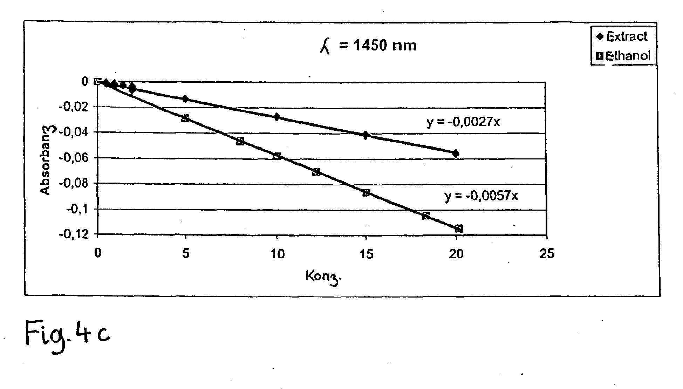

Die Fig. 4a bis 4c zeigen die Absorptions-Kennlinien von Alkohol, und Glukose, Fruktose und Saccharose, welche wesentliche Extraktbestandteile von Getränken sind, bei 1200 nm und 1300 nm. Es ist aus dieser Fig. 4a ersichtlich, dass bei einer Wellenlänge $\lambda$ = 1200 nm alle Komponenten bei jeweils gleicher Konzentration gleich absorbieren, bei $\lambda$ = 1300 nm und bei 1450 nm jedoch nicht (Fig. 4b, 4c). Dieser Unterschied im Absorptionsverhalten macht die Ethanol- und Extrakt-Konzentrations-Messung gemäß der Erfindung möglich.

Das Gleiche gilt auch für organische Säuren, wie Äpfel- und Weinsäure, sowie für Glyzerin, welche sich neben dem Zucker oft in Getränken befinden. Die Konzentrationskennlinien bei $\lambda$ = 1300 nm und $\lambda$ = 1450 nm sind beide linear und zeigen gleiche Charakteristik, daher ist es prinzipiell gleichwertig, ob man bei der Messung die Wellenlänge $\lambda$ = 1300 nm oder $\lambda$ = 1450 nm einsetzt. Ein Unterschied besteht bloß in der "Steilheit" der Kennlinien von Ethanol und Extrakt, welche bei $\lambda$ = 1450 nm etwa doppelt so hoch ist wie bei $\lambda$ = 1300 nm.

Die Genauigkeit bei der Bestimmung der Alkohol-Konzentration in einer denselben enthaltenden Probe wird durch Messung der Absorption von IR-Licht bei drei unterschiedlichen Wellenlängen, wie dies im Anspruch 2 vorgeschlagen ist, erhöht. Die Absorptionsmessung bei etwa $\lambda$ = 1300 nm und $\lambda$ = 1450 nm bringt im wesentlichen die gleiche Information, kann jedoch gerade bei trüben Proben für eine Trübungskorrektur günstig herangezogen werden.

Es können also durch Einsatz von IR-Licht mit wenigstens zwei, insbesondere mit drei, verschiedenen Wellenlängen rasch die Absorptionswerte der zu untersuchenden Probe bestimmt werden, wobei mittels einfacher Auswerteverfahren eine Auswertung der gemessenen Werte möglich ist und in weiterer Folge ein einfacher Vergleich mit vorher bestimmten Bezugswerten aus wenigstens einer Kalibriermessung vorgenommen werden kann.

Für eine einfache Auswertung der, gegebenenfalls gleichzeitig bei mehreren Wellenlängen festgestellten Werte für die Absorption von IR-Licht durch die zu untersuchende Probe wird erfindungsgemäß bevorzugt so vorgegangen, dass zur Bestimmung der Alkohol-Konzentration die für IR-Licht mit unterschiedlichen Wellenlängen erhaltenen Absorptionswerte mittels eines linearen Näherungsverfahrens, wie beispielsweise mittels linearer Regression, multilinearer Regression od. dgl., ausgewertet werden, und zwar unter Einbeziehung von im Rahmen der Kalibriermessung(en) erhaltenen Bezugswerten bzw. Konstanten, wobei bei Einsatz derartiger einfacher Näherungsverfahren auch ohne Zuhilfenahme von aufwendigen Auswerte- und Rechenverfahren das Auslangen gefunden werden kann.

Für eine weitere Erhöhung der Genauigkeit bei der Feststellung der Alkohol-Konzentration in einer flüssigen Probe wird darüber hinaus die Messung der Absorption bei konstanter Temperatur der zu untersuchenden Probe vorgenommen und/oder es wird die aktuelle Temperatur der zu untersuchenden Probe berücksichtigt.

Durch eine konstante Temperatur bzw. durch eine Thermostatisierung der Flüssigkeits-Probe lässt sich die Genauigkeit der Bestimmung der Alkohol-, insbesondere Ethanolkonzentration erhöhen bzw. es lässt sich bei Berücksichtigung der tatsächlichen Probentemperatur die Temperaturabhängigkeit der einzelnen Parameter ebenfalls berücksichtigen.

Zur Erzielung einer entsprechenden Genauigkeit bei der Bestimmung der Ethanolkonzentration, welche günstigerweise bei besser als 0,2 Vol.% und insbesondere bei 0,1 Vol.-% liegt, wird darüber hinaus bevorzugt vorgeschlagen, die Temperatur der Probe mit einer Genauigkeit von 0,1 °C, insbesondere < 0,05°C, einzustellen und bei dieser Temperatur zu messen.

Erfindungsgemäß wird als Lichtquelle eine NIR-LED eingesetzt. Die NIR-LED ist kostengünstig und hat fast unbegrenzte Lebensdauer, sie ist jedoch extrem temperaturempfindlich. Um die notwendige Genauigkeit und Stabilität der Messung der Alkoholkonzentration zu erreichen, ist es besonders günstig, die Temperatur der IR-Lichtquelle selbst zu stabilisieren. Dies kann beispielsweise mittels gezielten elektrischen Heizens erfolgen.

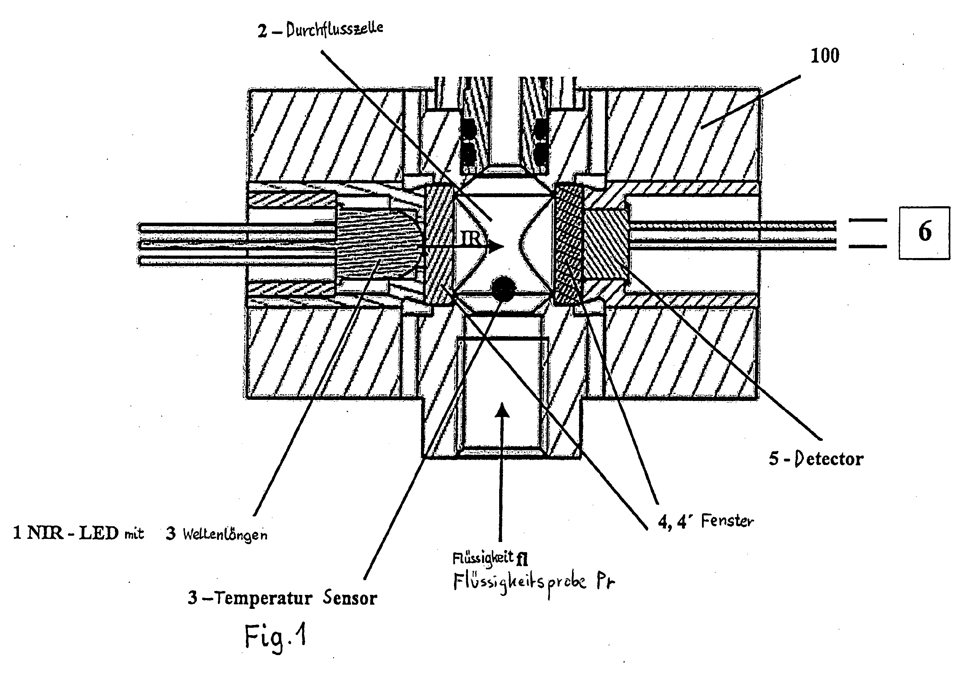

In der in Fig. 1 dargestellten neuen Vorrichtung 100, welche im Rahmen des neuen Verfahrens zur spektroskopischen Bestimmung der Konzentration von Alkoholen in flüssigen Proben eingesetzt werden kann, durchströmt die Flüssigkeit FI eine Durchflusszelle 2 für die Aufnahme der zu untersuchenden Probe, wobei als Lichtquelle 1 eine Leucht-Diode (LED) im nahen Infrarotbereich dient, welche IR-Strahlung mehrerer Wellenlängen abzustrahlen im Stande ist. Durch ein transparentes Fenster, z.B. ein spezielles Glasfenster 4, gelangt die elektromagnetische IR-Strahlung IR in die DurchflussZelle 1 mit der zu untersuchenden Flüssigkeits-Probe Pr und in weiterer Folge durch ein weiteres Fenster 4' zu dem Detektor 5. Weiters ist in der gezeigten Vorrichtung ein Sensor 3 für die Bestimmung der aktuellen Temperatur der Flüssigkeit FI und ein Sensor für die Kontrolle der Temperatur der LED 1 vorgesehen.

Der Detektor 5 unterscheidet zwischen den Wellenlängen, d.h. es können zumindest 2 oder sogar 3 Absorptionswerte gleichzeitig gemessen werden. Die Leuchtdiode wird elektrisch mit unterschiedlichen Anregungsfrequenzen moduliert. Das Signal vom Detektor 5 wird entsprechend diesen drei Frequenzen wieder demoduliert und auf diese Art können, praktisch gleichzeitig, drei Signale empfangen werden, die jeweils einer der Infrarotwellenlängen zuordenbar sind und die dann der Auswertungseinheit 6 zugeführt werden.

Selbstverständlich ist es auch möglich, dass die Wellenlängen-Unterscheidung in Zeitdomäne erfolgt, d.h., es wird nur mit einer von drei Leuchtdioden innerhalb eines bestimmtes Zeitintervalls gemessen, dann mit der zweiten LED, und schließlich mit der dritten.

Die Fig. 2 zeigt das Spektrum einer 3-farbigen NIR-LED, also einer LED, welche IR-Licht von drei verschiedenen Wellenlängen $\lambda 1$, $\lambda 2$ und $\lambda 3$ abstrahlen kann. Diese bevorzugt anzuwendende NIR-LED verfügt über einen integrierten Photodetekor, der eventuelle Schwankungen in der Lichtleistung der LED misst und so eine on line-Korrektur der Absorptionswerte der Proben zulässt.

Wird z.B. die Absorption nur bei einer Wellenlänge gemessen, könnte man zwischen Alkohol und Extrakt nicht unterscheiden. Bei einer Messung bei zwei oder mehr voneinander verschiedenen Wellenlängen ist dies möglich:

Die Absorption A ist, wie schon weiter oben kurz ausgeführt, im Wesentlichen proportional der Konzentration:

$$A = c1 \cdot Eps1 + c2 \cdot Eps2 + \ldots\ldots + cn \cdot Epsn,$$

worin c1, c2, ... cn die Konzentration der jeweiligen Stoffe 1, 2, ...n und Eps1, Eps2, ... Epsn die entsprechenden Absorptionskonstanten der Stoffe bedeuten.

Wenn die Stoff- bzw. Absorptionskonstanten von allen Komponenten in der Probe außer jener von Alkohol annähernd gleich sind und das ist der Fall, kann die Probe als System mit nur zwei Komponenten, bestehend aus Extrakt und Alkohol, behandelt werden.

Es gilt dann:

$$A1200 = Calk \cdot Epsalk1200 + Cext \cdot Epsext1200$$

$$A1300 = Calk \cdot Epsalk1300 + Cext \cdot Epsext1300$$

worin A1200 und A1300 die Gesamtabsorption bei $\lambda = 1200$ nm und $\lambda = 1300$ nm, Epsalk1200 und Epsalk1300 die Absorptions- (bzw. Extinktions-) Koeffizienten des Alkohols bei $\lambda = 1200$ und $\lambda = 1300$ nm, Epsext1200 und Epsext1300 die Absorptionskonstanten des Extraktes in der Flüssigkeitsprobe bei $\lambda = 1200$ und $\lambda = 1300$ nm, Cext die Konzentration des Extraktes in der Flüssigkeit und Calk die Konzentration des Alkohols in der Flüssigkeitsprobe bedeuten.

Die Stoffkonstanten Epsext1200 und Epsext1300 sind aus vorherigen Messungen an entsprechenden binären Lösungen ermittelt und somit bekannt. Es handelt sich hierbei um die Steigungen von Geraden, wie sie in der Fig. 4 gezeigt sind.

Es gilt dann für die Alkohol-Konzentration Calk einer Probe, bei Messung bei zwei unterschiedlichen Wellenlängen $\lambda = 1200$ und $\lambda = 1300$:

$$Calk = A + B \cdot A1200 + C' \cdot A1300$$

Die bei $\lambda = 1200$ und $\lambda = 1300$ nm erhaltenen Absorptionswerte A1200 und A1300 sind Messwerte, welche der Sensor bzw. die Sensoren 5 liefert bzw. liefern. Dann werden aus den obigen beiden Gleichungen mit zwei Unbekannten die beiden Konzentrationswerte Calk und Cext von Alkohol und Extrakt berechnet.

Im realen Fall wird zuerst die Lichtstreuung mittels Messung bei einer dritten Wellenlänge, also insbesondere im Bereich von $\lambda = 1450$ nm, kompensiert und erst dann wird, wie oben angegeben, gerechnet und ausgewertet.

In analoger Weise würde bei Absorptionsermittlung unter Einsatz von IR-Licht mit drei ausgewählten Wellenlängen drei unterschiedlichen mittels multilinearer bzw. multipler Regression gelten:

$$Calk = A + B \cdot A_{1200} + C' \cdot A_{1300} + D \cdot A_{1450} + E \cdot x$$

$E \cdot x$: Neben dem Einfluss des Extraktes bzw. der Extrakte können noch andere Komponenten in das Modell aufgenommen werden, wie insbesondere Dichte oder Farbwert.

Im Kalibrierversuch werden also jeweils Messungen von n Proben durchgeführt und die ermittelten Werte für die Konstanten A,B,C',D,E für die aus aufgrund der Ergebnisse von Referenzmethoden ermittelten Konzentrationen bestimmt, Anschließend wird das beschriebene Modell zur Ermittlung der Konzentrationen von bzw. in unbekannten Proben verwendet.

Das Gleichungssystem ist in bekannter Weise gemäß den in der multiplen Regression üblichen mathematischen Verfahren zu lösen, wobei gilt, je besser die Konstanten, umso genauer die Messung, was bedeutet, dass die erforderliche Genauigkeit hinsichtlich der Messung der Absorbance sinken kann, wodurch der Einsatz der einfachen "hand-held" IR-Absorptionsmessgeräte ermöglicht wird, die dann dennoch genügend exakte Werte liefern.

Der weiter oben besprochene binäre Ansatz mit nur zwei Wellenlängen gemäß den zwei allgemeinen Gleichungen

$$A1200 = Calk \cdot Epsalk1200 + Cext \cdot Epsext1200$$

$$A1300 = Calk \cdot Epsalk1300 + Cext \cdot Epsext1300$$

ist schon oben erklärt. Mit demselben sind die Messungen etwas weniger genau.

Es sei hier nur angemerkt, dass zu der Methode der multiplen Regression bzw. zur multivariablen Analyse eine große Anzahl von Literatur existiert, auf welche hier nicht näher eingegangen zu werden braucht.

In dem folgenden Beispiel wird die Erfindung näher erläutert:

Beispiel: In einer konkreten Testreihe mit einem Prototypen des verfahrensgemäß einzusetzenden neuen Absorptions-Messgerätes wurde der beschriebene einfache Aufbau um die Möglichkeit einer Temperierung der Probe erweitert.

Eine Durchflussküvette mit 7 mm optischer Weglänge wurde in einen Metallblock, der zwischen 10 und 40 °C temperierbar war, eingebaut, der gleichzeitig als Halter für die optischen Komponenten fungierte.

Auf einer Seite der Küvette wurde die NIR-LED montiert, auf der Gegenseite ein Ge - Detektor (EG&G, 3 mm Durchmesser) als Signaldetektor.

Als NIR-LEDs wurden drei Infrarot - LEDs in einem Gehäuse verwendet und mit modulierter Stromversorgung betrieben.

Zum Testen des Temperatureinflusses der LED erfolgte eine Heizung der LED mittels einer einfachen Widerstandsdraht-Wicklung um ihre Kappe. Dieser Testparameter sowie der Temperierblock sind in der wie oben beschriebenen Ausführung nicht vorgesehen, da hier die Vorgabe einer besonders preiswerten und handlichen Ausführungsform gegeben ist und entsprechende Referenzmessungen mittels Standardlösungen bzw. Kalibrierung hinreichend genaue Ergebnisse erbringen.

Dabei wurde der Stromfluss so gewählt, dass gut detektierbare Signale sowohl am IR - Detektor als auch am Referenz-Detektor erhalten wurden. Es wurde mit Frequenzen von 170, 180 und 190 Hz moduliert.

Es handelt sich bei der lichttechnischen Ausrüstung um die Anordnung Epitex multiwavelength LED L1200/1300/1450/PD-35B32 mit integriertem Referenzdetektor.

Die detektierten Photoströme wurden in demodulierte Spannungswerte übersetzt (trans-Impedanzverstärker und synchron demodulierender lock-in), und die gesammelten Daten wurden elektronisch (mittels PC-Karte und LabView Software) weiterverarbeitet. Gemessen wurde eine Vielzahl realer Proben, einerseits mit Referenzlaborgeräten der Type Alkolyzer Plus mit ± 0,1 %Vol/Vol Genauigkeit) als auch mit dem - wie hier beschriebenen - Testaufbau des Absorptions-Messgerätes gemäß der Erfindung.

Die Messung erfolgte über die abwechselnde Messung von Wasser und Probe und Korrektur bzw. Abgleich mit der Wasserreferenz (c=0)

Der Extrakt einer Probe kann unter Verwendung der bekannten Tabarie - Formel aus Ethanol-Konzentration und Dichte berechnet werden.

Exemplarisch werden hier die grundsätzlichen Ergebnisse von sieben von insgesamt 24 auf ihren Alkoholgehalt untersuchten österreichischen und ausländischen Weinproben tabellarisch dargestellt; wobei in den Spalten nacheinander die Proben- Nr. , der untersuchte Wein mit Herkunftsangabe, der mit einem handelsüblichen, komplizierten und teuren Gerät ermittelte Ethanolgehalt, der Gehalt an Extrakt und schließlich die auf erfindungsgemäße Weise einfach und billig nur auf Grund von Absorptionsmessungen und auf Grund von Absorptionsmessungen unter Einbeziehung der Dichte der Proben ermittelten Prozentgehalte an Ethanol in den untersuchten Proben angegeben sind.

**Tabelle 1: Liste der Wein-Proben**

| Nr. | Probe, Herkunft | Ethanolgehalt, ermittelt mit Alcolyzer (% Vol/Vol) | Extract (%Gew/Vol) | % Ethanol, *) ermittelt mit Absorption % % Vol/Vol | % Ethanol, **) ermittelt mit Abs+Dichte % Vol/Vol |
|---|---|---|---|---|---|
| 2 | Merlot, Frankreich | 12,37 | 2,80 | 12.36 | 12.47 |
| 4 | Welschriesling Trunk 2003 | 12,43 | 1,65 | 12.29 | 12.38 |
| 5 | Galser Spätlese Cuvée Österr. | 10,73 | 8,03 | 10.82 | 10.71 |
| 11 | Vino Tinto, Spanien | 10,57 | 6,84 | 10.51 | 10.55 |
| 12 | Blauer Zweigelt 2003, Österr. | 12,92 | 3,15 | 12.87 | 12.78 |
| 14 | Portugieser Weißherbst 2002, Osterr. | 8,80 | 5,92 | 9.03 | 8.84 |
| 21 | Ruby Cabernet | 12,87 | 3,52 | 13.28 | 12.88 |
| *) gemessen bei 3 unterschiedlichen Wellenlängen **) gemessen bei 3 unterschiedlichen Wellenlängen | | | | | |

Es wurden also insgesamt 24 Weinsorten (mit 9 bis 13,3 Vol-% Alkohol) und 28 Biersorten (mit 0 bis 9,6 Vol% Alkohol) und unterschiedlichsten Extraktwerten mit einem Prototypen des neuen Handheld-Gerätes (bei $\lambda$ = 1180 nm ("$\lambda$ 1200"), $\lambda$ = 1295 nm $\lambda$ ("$\lambda$1300) und $\lambda$ = 1430 nm ("$\lambda$ 1450") gemessen, gleichzeitig wurden für die untersuchten Proben mit Referenzmethoden folgende Größen charakterisiert:

Im Versuch wurden jeweils Messungen von n-1 Proben durchgeführt und die ermittelten Werte für die Konstanten A,B,C', D,E für die Konzentrationsbestimmung der n-ten Probe verwendet. Dann wurde verglichen, wie der Konzentrationswert der n-ten Probe mit dem tatsächlichen Wert aus der Referenzbestimmung übereinstimmt.

Alkoholgehalt: Der bekannte Alcolyzer verwendet den spezifischen Absorptionspeak bei 1180 nm zur Konzentrationsbestimmung, die Messung erfolgt in thermostatisierter Küvette und die Wellenlängeneinstellung mit einem Gitterspektrometer

Dichtemessung mit einem DMA4500, Durchfluss-Dichtemessgerät der Firma Paar, Österreich (linear mit dem Extraktwert der Probe korreliert)

Farbmessung (bei den Bierproben)

Die Messungen für insgesamt 24 Weinproben und 28 Biersorten wurden statistisch ausgewertet, es ergaben sich für die untersuchten Probenauswahlen folgende Werte des Standard Error of Cross Validation (SECV).

Der SECV errechnet sich bei der statistischen Evaluierung der Modelle aus der Summe der Quadrate der Abweichungen vom tatsächlichen Referenzwert

Für die vermessenen Weinproben mit den Absorbances A1200, A1300, A1450 bei $\lambda$ 1 = etwa 1200 nm, $\lambda$2 = etwa 1300 nm, $\lambda$3 = etwa 1450 nm und evtl. zusätzlichen Parametern x im Regressionsmodell ergibt sich:

| Proben | Modellparameter, gemessene bzw. berücksichtigte Größen | SECV (%Vol/Vol) |
|---|---|---|
| Wein | Absorption | 0.28 |
| Wein | Absorption und Leitfähigkeit | 0.16 |
| Wein | Absorption und Dichte | 0.09 |
| Bier | Absorption, Dichte und Farbe | 0.06 |

Es ist hier zum ersten Mal die Kombination von zwei Absorptionswerten bei verschiedenen IR-Wellenlängen in an sich breit "verschmierten" - hier von LED-IR-Quellen stammenden - Spektren vorgeschlagen. Die beiden ersten IR-Wellenlängen liegen bei λ = etwa 1200 und λ = etwa 1300 nm, das sind zwei Wellenlängen-Bereiche, durch welche die Auftrennung in Alkohol und Extrakt ermöglicht wird.

Bei Kombination der Ermittlung bei den beiden ersten IR-Wellenlängen mit der Absorptionsmessung bei einer dritten IR-Wellenlänge λ3 = etwa 1450 nm werden zusätzlich Streulicht-Anteile berücksichtigt und auf diese Weise werden die Genauigkeit und Richtigkeit der Messung erhöht.

**Patentansprüche**

1. Verfahren zur Ermittlung des Gehaltes an Ethanol oder eines anderen Alkohols von zumindest Wasser und Ethanol oder einen anderen Alkohol enthaltenden Flüssigkeiten (Fl) bzw. Flüssigkeitsproben (Pr), insbesondere von Getränken, Arzneimitteln, Kosmetika u.dgl., sowie mindestens eines weiteren Gehaltes der Flüssigkeit (Fl) an Extraktstoffen und/oder Zuckern und/oder Fruchtsäure od. dgl., wobei die sich in einer Analysenzelle (2) befindliche oder eine Durchflusszelle (2) durchströmende Flüssigkeit (Fl) von zumindest einer mit einer LED (1) ausgebildeten Lichtquelle, welche IR-Strahlung mit einer Wellenlänge im Bereich von 900 bis 1500 nm abgibt, durchstrahlt wird, **dadurch gekennzeichnet,**

   - **dass** die IR-Lichtabsorption von breitbandiger IR-Strahlung der zumindest einer LED(1), deren Peakmaxima bei zumindest zwei unterschiedlichen Wellenlängen liegen, ohne Zwischenschaltung einer Monochromator-Einrichtung, gemessen wird und die Messwerte auf elektronischem Weg, in Angaben zumindest des Alkoholgehaltes der Flüssigkeit (FL) umgewandelt werden, (Fl)
   - wobei die Flüssigkeit (Fl) entweder zeitlich nacheinander oder praktisch gleichzeitig, mit einer ersten breitbandigen IR-Strahlung deren Peakmaximum bei einer ersten Wellenlänge λ1 liegt, bei welcher der Absorptionskoeffizient des Ethanols oder anderen Alkohols Epsalkλ1 und der Absorptionskoeffizient des Wassers Epswλ1 zumindest im Wesentlichen untereinander gleich groß sind,
   - und mit zumindest einer zweiten breitbandigen IR-Strahlung, deren Peakmaximum bei einer zweiten Wellenlänge λ2 liegt, bei welcher der Absorptionskoeffizient des Ethanols oder anderen Alkohols Epsalkλ2 und der Absorptionskoeffizient des Wassers Epswλ2 möglichst verschieden voneinander sind, durchstrahlt wird,
   - wobei die eingesetzten Peaks der IR-Strahlung eine Halbwertsbreite von 50 bis 100 nm aufweisen, und
   - **dass** die jeweils zumindest zwei mittels zumindest einem IR-Detektor (5) aktuell ermittelten, realen Absorptions-Messwerte unter Nutzung des linearen Zusammenhangs zwischen Alkoholkonzentration der Flüssigkeit (Fl) und der Absorption des IR-Lichtes durch die Flüssigkeit (Fl) bei der jeweiligen Strahlung einer Rechen- und Ausgabeeinheit (6) für Berechnung und Anzeige bzw. Ausgabe zumindest des Alkoholgehaltes der Flüssigkeit (Fl) zugeführt werden.

2. Verfahren nach Anspruch 1, **dadurch gekennzeichnet, dass** die breitbandige IR-Strahlung mit einem Peakmaximum bei einer zweiten Wellenlänge λ2 eingesetzt wird, bei welcher der Absorptionskoeffizient des Ethanols oder anderen Alkohols Epsalkλ2 sehr unterschiedlich ist zum, bzw. höher ist als der, Absorptionskoeffizient des Wassers Epswλ2.

3. Verfahren nach Anspruch 1 oder 2, **dadurch gekennzeichnet, dass**, insbesondere zur Erhöhung der Genauigkeit des Ergebnisses bezüglich des Gehaltes an Ethanol oder anderem Alkohol und/oder zur Kompensation von Trübungen in der Flüssigkeit (Fl) un/oder zur Ermittlung des Gehaltes der Flüssigkeit (Fl) an anderen Komponenten als Ethanol oder anderem Alkohol, wie insbesondere Zucker, Extrakte, Farbstoffe, und/oder Fruchtsäuren, die zu untersuchende Flüssigkeit (Fl) mit einer dritten breitbandigen IR-Strahlung mit einem Peakmaximum bei einer dritten Wellenlänge λ3 durchstrahlt wird, - wobei deren Peak eine Halbwertsbreite von 50 bis 100 nm aufweist - bei welcher Wellenlänge λ3 der Absorptionskoeffizient des Alkohols Epsalkλ3 klar unterschiedlich ist zum, gegebenfalls höher ist als der, Absorptionskoeffizient des Wassers Epswλ3.

4. Verfahren nach einem der Ansprüche 1 bis 3, **dadurch gekennzeichnet, dass** eine breitbandige IR-Strahlung eingesetzt wird, deren Peaks jeweils eine Halbwertsbreite von 70 bis 90 nm aufweisen.

5. Verfahren nach einem der Ansprüche 1 bis 4, **dadurch gekennzeichnet, dass** die zu untersuchende Flüssigkeit (Fl) mit einer ersten breitbandigen IR-Strahlung mit einem Peakmaximum bei einer ersten Wellenlänge λ1 durchstrahlt wird, bei welcher die im wesentlichen gleich hohen, Absorptionskoeffizienten von Ethanol oder dem anderen Alkohol Epsalkλ1 und von Wasser Epswλ1 jeweils im plus-bis-minus-Nah-Bereich des Maximums oder am Maximum eines Peaks in den beiden Wellenlängen-Absorptionskoeffizienten-Diagrammen bzw. -Funktionen von Ethanol oder den anderen Alkoholen und Wasser liegen.

**6.** Verfahren nach einem der Ansprüche 1 bis 5 **dadurch gekennzeichnet, dass** die zu untersuchende Flüssigkeit (Fl) mit einer zweiten und/oder dritten breitbandigen IR-Strahlung mit einem Peakmaximum bei einer zweiten Wellenlänge $\lambda 2$ und/oder bei einer dritten Wellenlänge $\lambda 3$ durchstrahlt wird, bei welcher die Absorptionskoeffizienten von Ethanol oder anderem Alkohol Epsalk$\lambda 2$ und/oder Epsalk$\lambda 3$ und von Wasser Epsw$\lambda 2$ und/oder Epsw$\lambda 3$ voneinander möglichst verschieden sind und jeweils im plus-bis-minus-Nah-Bereich des Maximums oder am Maximum eines Peaks in den beiden Wellenlängen-Absorptionskoeffizienten-Diagrammen bzw. -Funktionen von Ethanol oder anderem Alkohol und Wasser liegen.

**7.** Verfahren nach einem der Ansprüche 1 bis 6, **dadurch gekennzeichnet, dass** eine breitbandige IR-Strahlung aus einer LED-IR-Lichtquelle (1), eingesetzt wird, welche gegebenenfalls gleichzeitig, zwei, gegebenenfalls drei, unterschiedliche, den Kriterien der vorangegangenen Ansprüchen entsprechende Peaks bei Wellenlängen $\lambda 1$, $\lambda 2$, und $\lambda 3$, emittiert, wobei die Peakmaxima der IR-Strahlung im Bereich
von $\lambda 1$ bei 1170 bis 1190 nm, bevorzugt bei 1180 nm ("$\lambda 1200$")
von $\lambda 2$ bei 1290 bis 1300 nm, bevorzugt bei 1295 nm ("$\lambda 1300$") und
von $\lambda 3$ von 1425 bis 1435 nm, bevorzugt bei 1430 nm ("$\lambda 1450$"), liegen.

**8.** Verfahren nach einem der Ansprüche 1 bis 7, **dadurch gekennzeichnet, dass**, mittels einer der LED-IR-Lichtquelle (1) zugeordneten bzw. in dieselbe integrierten Lichtintensitäts-Messeinheit, vorzugsweise mittels Photodetektor, Lichtintensitäts-Schwankungen der IR-Strahlung kompensiert bzw. korrigiert werden.

**9.** Verfahren nach einem der Ansprüche 1 bis 8, **dadurch gekennzeichnet, dass** zusätzlich zur Messung der IR-Licht-Absorption auch die Dichte und/oder die elektrische Leitfähigkeit der Flüssigkeit (Fl) gemessen und unter Einbeziehung der Ergebnisse von entsprechenden Vergleichs- bzw. Kalibriermessungen in die Auswertung mit einbezogen wird.

**10.** Verfahren nach Anspruch 9, **dadurch gekennzeichnet, dass** zur Bestimmung der Alkohol-Konzentration Calk in der Flüssigkeitsprobe (Pr) die für die IR-Strahlung bei den unterschiedlichen Wellenlängen erhaltenen aktuellen IR-Licht-Absorptionswerte A$\lambda 1$, A$\lambda 2$, A$\lambda 3$ sowie weiters die Werte von Dichte und/oder Leitfähigkeit der Flüssigkeit mittels eines linearen Näherungsverfahrens, wie insbesondere mittels linearer Regression, multilinearer oder multipler Regression, ausgewertet werden, und zwar unter Verwendung der entsprechenden, durch vorherige Kalibriermessungen ermittelten Bezugswerte.

**11.** Verfahren nach einem der Ansprüche 1 bis 10, **dadurch gekennzeichnet, dass** während der Messung die Temperatur der die Durchflusszelle (2) durchströmenden Probe (Pr) konstant gehalten wird, dass also die Bestimmung der IR-Licht-Absorption der zu untersuchenden Flüssigkeit (Fl) bei konstanter Temperatur derselben vorgenommen wird oder dass die aktuell gemessene Temperatur der zu untersuchenden Flüssigkeit (Fl) bei der Auswertung der Messergebnisse mit berücksichtigt wird oder elektronisch mittels Software kompensiert wird oder dass die Temperatur der IR-Lichtquelle (1) zumindest während der Durchstrahlung der Flüssigkeit (Fl) und der Bestimmung des Wertes von deren realer Absorption konstant gehalten wird.

**12.** Vorrichtung (100) zur Durchführung des Verfahrens nach einem der Ansprüche 1 bis 11 mit einer die zu untersuchende, Ethanol oder zumindest einen anderen Alkohol enthaltende wässrige Flüssigkeit (Fl) aufnehmenden bzw. beinhaltenden Analysenzelle oder einer von derselben zu durchströmenden Durchflusszelle (2), mit einem Temperatursensor (3) für die die Ermittlung der aktuellen Flüssigkeitstemperatur und/oder Mitteln zur Temperierung der Flüssigkeit (Fl) oder Konstanthaltung der Probentemperatur und zwei einander gegenüberliegenden, für IR-Strahlung durchgängigen Fenstern (4, 4') und einer entweder gleichzeitig oder jeweils sequenziell breitbandige IR-Strahlung mit zumindest zwei bei unterschiedlichen Wellenlängen $\lambda 1$, $\lambda 2$ liegenden Peakmaxima oder mit drei bei zueinander unterschiedlichen Wellenlängen $\lambda 1$, $\lambda 2$, $\lambda 3$, liegenden Peakmaxima und jeweils Peaks mit einer Halbwertsbreite von 50 bis 100 nm, insbesondere von 70 bis 90 nm, emittierenden Infrarot-LED-Strahlungsquelle (1) - ohne Zwischenschaltung einer Monochromator-Einrichtung hinter einem der Fenster (4) - und einem IR-Strahlungsdetektor (5) hinter dem anderen Fenster (4'), und mit einer Recheneinheit (6) zur Verarbeitung der von demselben ermittelten Absorptions-Messwerte (A) sowie mit einer Ausgabeeinheit zur Anzeige oder Ausgabe zumindest des Gehaltes an Ethanol oder einem anderen Alkohol in der Flüssigkeit (Fl), wobei der IR-Strahlungsdetektor (5) mit der Recheneinheit (6) und der Ausgabeeinheit verbunden ist.

**13.** Vorrichtung nach Anspruch 12, **dadurch gekennzeichnet, dass** der Infrarot-LED-Strahlungsquelle (1) zur Kompensation bzw. Korrektur von Schwankungen der Intensität der von derselben abgegebenen Strahlung, eine Strahlungsintensitäts-Messeinheit, insbesondere ein Photodetektor, zugeordnet bzw. in oder an dieselbe integriert ist, welcher mit der Recheneinheit (6) datenfluss-verbunden ist.

**Claims**

1. A method for determining the content of ethanol or or another alcohol of at least water and ethanol or liquids (Fl) or liquid samples (Pr) containing another alcohol, in particular of drinks, pharmaceuticals, cosmetics, and the like, as well as at least an additional content of the liquid (Fl) of extracts and/or sugar and/or fruit acid or the like, wherein the liquid (Fl) located in an analysis cell (2) or flowing through a flow-through cell is irradiated by at least one light source designed with a LED (1), which emits radiation with a wave length in the range of 900 to 1500 nm, **characterized in,**

   - **that** the IR (infrared) light absorption of broadband IR radiation of the at least one LED (1), the peak maxima of which are at at least two different wave lengths, is measured without interconnection of a monochromator device and the measured values are converted by electronic means into data at least of the alcohol content of the liquid (Fl),
   - wherein the liquid (Fl) is irradiated either chronologically successively or practically simultaneously, with a first broadband IR radiation the peak maximum of which lies at a first wave length $\lambda 1$, at which the absorption coefficient of the ethanol or other alcohol Epsalk $\lambda 1$ and the absorption coefficient of the water Epsw$\lambda 1$ are at least substantially commensurate among themselves,
   - and is irradiated with at least a second broadband IR radiation, the peak maximum of which lies at a second wave length $\lambda 2$, at which the absorption coefficient of the ethanol or other alcohol Epsalk $\lambda 2$ and the absorption coefficient of the water Epsw$\lambda 2$ are as different from each other as possible,
   - wherein the peaks of the IR radiation employed have a peak-width at half height of 50 to 100 nm, and
   - **that** each of the at least two real absorption measured values currently determined by means of at least one IR-detector (5) by using the linear relationship between alcohol concentration of the liquid (Fl) and the absorption of the IR light by the liquid (Fl) in the case of the respective radiation are supplied to a computing- and output unit (6) for calculation and display or output at least of the alcohol content of the liquid (Fl).

2. A method according to claim 1, **characterized in that** the broadband IR radiation is used with a peak maximum at a second wave length $\lambda 2$, in which the absorption coefficient of the ethanol or other alcohol Epsalk$\lambda 2$ is very different from or is higher than the absorption coefficient of the water Epsw$\lambda 2$.

3. A method according to claim 1 or 2, **characterized in that**, particularly to increase the precision of the result with regard to the content of ethanol or other alcohol and/or to compensate for turbidities in the liquid (Fl) and/or to determine the content of the liquid (Fl) of other components than ethanol or other alcohol, such as particularly sugar, extracts, dyes, and/or fruit acids, the liquid (Fl) to be examined is irradiated with a third broadband IR radiation with a peak maximum at a third wave length $\lambda 3$, - wherein its peak has a peak-width at half height of 50 to 100 nm - in which wave length $\lambda 3$ of the absorption coefficient of the alcohol Epsalk$\lambda 3$ is clearly different from, if necessary, higher than the absorption coefficient of the water Epsw$\lambda 3$.

4. A method according to one of claims 1 to 3, **characterized in that** a broadband IR radiation is used, the peaks of which in each case have a peak-width at half height of 70 to 90 nm.

5. A method according to one of claims 1 to 4 **characterized in that** the liquid (Fl) to be examined is irradiated with a first broadband IR radiation with a peak maximum at a first wave length $\lambda 1$, in which the essentially equally high absorption coefficients of ethanol or the other alcohol Epsalk $\lambda 1$ and of water Epsw$\lambda 1$ in each case lie in the plus-to-minus range of the maximum or at the maximum of a peak in both wave length-absorption coefficient diagrams or functions of ethanol or the other alcohols and water.

6. A method according to one of claims 1 to 5 **characterized in that** the liquid (Fl) to be examined is irradiated with a second and/or third broadband IR radiation with a peak maximum at a second wave length $\lambda 2$ and/or at a third wave length $\lambda 3$, in which the absorption coefficients of ethanol or other alcohol Epsalk $\lambda 2$ and/or Epsalk $\lambda 3$ and of water Epsw$\lambda 2$ and/or Epsw$\lambda 3$ are as different from each other as possible and in each case lie in the plus-to-minus range of the maximum or at the maximum of a peak in both wave length-absorption coefficient diagrams or functions of ethanol or the other alcohols and water.

7. A method according to one of claims 1 to 6, **characterized in that** a broadband IR radiation from a LED-IR light source (1) is used, which if necessary simultaneously emits two, if necessary three, different peaks corresponding to the criteria of the preceding claims at wave lengths λ1, λ2, and λ3, wherein the peak maxima of the IR radiation lie in the range
of λ1 at 1170 to 1190 nm, preferably at 1180 nm ("λ1200")
of λ2 at 1290 to 1300 nm, preferably at 1295 nm ("λ1300") and
of λ3 at 1425 to 1435 nm, preferably at 1430 nm ("λ1450").

**8.** A method according to one of claims 1 to 7, **characterized in that** by means of a light intensity measuring unit assigned to the LED IR light source (1) or integrated into the same, preferably by means of a photo-detector, light intensity fluctuations of the IR radiation are compensated for or corrected.

**9.** A method according to one of claims 1 to 8, **characterized in that** in addition to the measurement of the IR light absorption the density and/or the electrical conductivity of the liquid (Fl) is measured and taking into account the results of relevant comparison or calibration measurements is included in the evaluation.

**10.** A method according to claim 9, **characterized in that** to determine the calk alcohol concentration in the liquid sample (Pr) the current IR light absorption values Aλ1, Aλ2, Aλ3 obtained for the IR radiation at the different wave lengths as well as also the values of density and/or conductivity of the liquid by means of a linear approximation method, such as in particular by means of linear regression, multi-linear or multiple regression, are evaluated, specifically by using the relevant reference values determined by previous calibration measurements.

**11.** A method according to one of claims 1 to 10, **characterized in that** during the measurement the temperature of the sample (Pr) flowing through the through-flow cell (2) is kept constant, that therefore the determination of the IR light absorption of the liquid (F1) to be examined is carried out at the constant temperature of the same or that the current measured temperature of the liquid (Fl) to be examined is taken into consideration during the evaluation of the measurement results or is compensated for electronically by means of software or that the temperature of the IR light source (1) is kept constant at least during the irradiation of the liquid (Fl) and the determination of the value of its real absorption.

**12.** A device (100) for implementing the method according to one of claims 1 to 11 with an analysis cell receiving or containing watery liquid (F1) to be examined containing ethanol or at least one other alcohol or a through-flow cell (2) to be flowed through by the same, with a temperature sensor (3) for determining the current liquid temperature and/or means for tempering the liquid (Fl) or keeping the sample temperature constant and two continuous windows (4, 4') opposite each other for IR radiation and an either simultaneous or in each case sequential broadband IR radiation with at least two peak maxima lying at different wave lengths λ1, λ2 and each infrared LED radiation source (1) emitting peaks with a peak-width at half height of 50 to 100 nm, in particular of 70 to 90 nm, - without interconnection of a monochromator device behind one of the windows (4) - and an IR radiation detector (5) behind the other window (4'), and with a computing unit (6) for processing the absorption measured values (A) determined by the same as well as with an output unit for the display or output of at least the content of ethanol or another alcohol in the liquid (Fl), wherein the IR radiation detector (5) is connected to the computing unit (6) and the output unit.

**13.** A device according to claim 12, **characterized in that** the infrared LED radiation source (1) for compensating for or correcting the fluctuations of the intensity of the radiation emitted by the same, is assigned a radiation intensity measuring unit, in particular a photo-detector or is integrated into or to the same, which is connected in terms of data-flow with the computing unit (6).

**Revendications**

**1.** Procédé pour déterminer la teneur en éthanol ou un autre alcool dans des liquides (Fl), contenant au moins de l'eau et de l'éthanol ou un autre alcool, ou dans des échantillons de liquides (Pr), en particulier des boissons, des produits pharmaceutiques, des cosmétiques, etc., ainsi qu'au moins une autre teneur du liquide (Fl) en extraits et/ou sucres et/ou acides de fruit ou similaires, le liquide (Fl) contenu dans une cellule d'analyse (2) ou circulant à travers une cellule de passage (2) étant traversé par le rayonnement d'au moins une source lumineuse, réalisée avec une diode électroluminescente (1) et émettant un rayonnement infrarouge avec une longueur d'onde dans une plage de 900 à 1500 nm, **caractérisé**

- **en ce que** l'absorption de lumière infrarouge par un rayonnement infrarouge à large bande de ladite au moins

une diode electroluminescente (1), dont les maxima du pic se situent à au moins deux longueurs d'onde différentes, est mesurée sans que soit intercalé un monochromateur, et les valeurs de mesure sont transformées par voie électronique en données indiquant au moins la teneur en alcool du liquide (Fl),

- le liquide (Fl) étant traversé, soit successivement dans le temps ou pratiquement simultanément, par un premier rayonnement infrarouge à large bande dont le maximum du pic se situe à une première longueur d'onde λ1, à laquelle le coefficient d'absorption de l'éthanol ou d'un autre alcool Epsalkλ1 et le coefficient d'absorption de l'eau Epswλ1 sont au moins sensiblement identiques l'un à l'autre,

- et par au moins un deuxième rayonnement infrarouge à large bande dont le maximum du pic se situe à une deuxième longueur d'onde λ2, à laquelle le coefficient d'absorption de l'éthanol ou d'un autre alcool Epsalkλ2 et le coefficient d'absorption de l'eau Epswλ2 sont si possible différents l'un de l'autre,

- les pics utilisés du rayonnement infrarouge comportant une largeur à mi-hauteur de 50 à 100 nm, et

- **en ce que**, moyennant l'utilisation de la corrélation linéaire entre la concentration d'alcool du liquide (Fl) et l'absorption de la lumière infrarouge à travers le liquide (Fl), les respectivement au moins deux valeurs de mesure d'absorption réelles, déterminées actuellement au moyen d'au moins un détecteur infrarouge (5), sont acheminées lors du rayonnement respectif vers une unité de calcul et d'affichage (6) pour calculer et afficher ou éditer au moins la teneur en alcool du liquide (Fl).

**2.** Procédé selon la revendication 1, **caractérisé en ce que** le rayonnement infrarouge à large bande avec un maximum du pic est utilisé à une deuxième longueur d'onde λ2, à laquelle le coefficient d'absorption de l'éthanol ou d'un autre alcool Epsalkλ2 est très différent, plus précisément plus élevé que le coefficient d'absorption de l'eau Epswλ2.

**3.** Procédé selon la revendication 1 ou 2, **caractérisé en ce que**, en particulier pour augmenter la précision du résultat relatif à la teneur en éthanol ou un autre alcool et/ou pour compenser les troubles dans le liquide (Fl) et/ou pour déterminer la teneur du liquide (Fl) en composants différents de l'éthanol ou d'un autre alcool, tels que le sucre, les extraits, les colorants et/ou acides de fruits, le liquide (Fl) à examiner est traversé par un troisième rayonnement infrarouge à large bande avec un maximum du pic à une troisième longueur d'onde λ3 - dont le pic comporte une largeur à mi-hauteur de 50 à 100 nm - à laquelle troisième longueur d'onde λ3, le coefficient d'absorption de l'éthanol ou d'un autre alcool Epsalkλ3 est nettement différent, le cas échéant, plus élevé que le coefficient d'absorption de l'eau Epswλ3.

**4.** Procédé selon l'une quelconque des revendications 1 à 3, **caractérisé en ce que** l'on utilise un rayonnement infrarouge à large bande dont les pics comportent chacun une largeur à mi-hauteur de 70 à 90 nm.

**5.** Procédé selon l'une quelconque des revendications 1 à 4, **caractérisé en ce que** le liquide (Fl) à examiner est traversé par un premier rayonnement infrarouge à large bande avec un maximum du pic à une première longueur d'onde λ1, à laquelle le coefficient d'absorption de l'éthanol ou d'un autre alcool Epsalkλ1 et le coefficient d'absorption de l'eau Epswλ1, sensiblement de même valeur, se situent respectivement dans une zone plus ou moins proche du maximum ou au maximum d'un pic dans les deux diagrammes ou fonctions longueur d'onde - coefficients d'absorption de l'éthanol ou des autres alcools et de l'eau.

**6.** Procédé selon l'une quelconque des revendications 1 à 5, **caractérisé en ce que** le liquide (Fl) à examiner est traversé par un deuxième et/ou troisième rayonnement infrarouge à large bande avec un maximum du pic à une deuxième longueur d'onde λ2 et/ou à une troisième longueur d'onde λ3, à laquelle les coefficients d'absorption de l'éthanol ou d'un autre alcool Epsalkλ2 et/ou Epsalkλ3 et de l'eau Epswλ2 et/ou Epswλ3 sont si possible différents l'un de l'autre et se situent respectivement dans une zone plus ou moins proche du maximum ou au maximum d'un pic dans les deux diagrammes ou fonctions longueur d'onde - coefficients d'absorption de l'éthanol ou d'un autre alcool et de l'eau.

**7.** Procédé selon l'une quelconque des revendications 1 à 6, **caractérisé en ce que** l'on utilise un rayonnement infrarouge à large bande provenant d'une source lumineuse infrarouge à diode électroluminescente (1) qui émet, le cas échéant, simultanément deux, le cas échéant, trois pics différents correspondant aux critères des revendications précédentes à des longueurs d'onde λ1, λ2 et λ3, les maxima du pic du rayonnement infrarouge se situant dans une plage

pour λ1 de 1170 à 1190 nm, de préférence à 1180 nm ("λ1200"),

pour λ2 de 1290 à 1300 nm, de préférence à 1295 nm ("λ1300"), et

pour λ3 de 1425 à 1435 nm, de préférence à 1430 nm ("λ1450").

**8.** Procédé selon l'une quelconque des revendications 1 à 7, **caractérisé en ce que** les variations d'intensité lumineuse du rayonnement infrarouge sont compensées ou corrigées au moyen d'une unité de mesure de l'intensité lumineuse, associée à la source lumineuse infrarouge à diode électroluminescente (1) ou intégrée dans celle-ci, de préférence au moyen d'un photodétecteur.

9. Procédé selon l'une quelconque des revendications 1 à 8, **caractérisé en ce que**, en plus de la mesure de l'absorption de la lumière infrarouge, on mesure également la densité et/ou la conductibilité électrique du liquide (FI) qui sont incluses dans l'analyse moyennant l'intégration des résultats des mesures de comparaison ou de calibrage correspondantes.

10. Procédé selon la revendication 9, **caractérisé en ce que** pour déterminer la concentration en alcool Calk dans l'échantillon de liquide (Pr), les valeurs actuelles de l'absorption de lumière infrarouge A$\lambda$1, A$\lambda$2, A$\lambda$3, obtenues aux différentes longueurs d'onde, ainsi que, par ailleurs, les valeurs de densité et/ou de conductibilité du liquide sont analysées au moyen d'un procédé d'approximation linéaire, comme, en particulier, au moyen d'une régression linéaire, d'une régression multilinéaire ou multiple, à savoir moyennant l'utilisation des valeurs de référence correspondantes, déterminées par les mesures de calibrage précédentes.

11. Procédé selon l'une quelconque des revendications 1 à 10, **caractérisé en ce que** pendant la mesure, la température de l'échantillon de liquide (Pr) circulant à travers la cellule de passage (2) est maintenue constante, **en ce que**, par conséquent, l'absorption de la lumière infrarouge du liquide (FI) à examiner est déterminée à une température constante de celui-ci ou **en ce que** la température mesurée actuellement du liquide (FI) à examiner est prise en compte lors de l'analyse des résultats de mesure ou est compensée électroniquement au moyen d'un logiciel, ou **en ce que** la température de la source lumineuse infrarouge (1) est maintenue constante au moins pendant le passage du rayonnement à travers le liquide (FI) et pendant la détermination de la valeur de l'absorption réelle de ce liquide.

12. Dispositif (100) pour la mise en oeuvre du procédé selon l'une quelconque des revendications 1 à 11, comportant une cellule d'analyse, recevant ou contenant le liquide (FI) aqueux à examiner, qui contient de l'éthanol ou au moins un autre alcool, ou une cellule de passage (2) traversée par ce même liquide, comportant un capteur de température (3) pour déterminer la température actuelle du liquide et/ou des moyens pour réguler la température du liquide (FI) ou pour maintenir constante la température de l'échantillon, et deux fenêtres (4, 4') face à face laissant passer le rayonnement infrarouge, et une source lumineuse infrarouge à diode électroluminescente (1) émettant simultanément ou séquentiellement un rayonnement infrarouge à large bande avec au moins deux maxima du pic situés à des longueurs d'onde $\lambda$1, $\lambda$2 différentes ou avec trois maxima du pic situés à des longueurs d'onde $\lambda$1, $\lambda$2, $\lambda$3 différentes et respectivement des pics avec une largeur à mi-hauteur de 50 à 100 nm, en particulier de 70 à 90 nm - sans le montage intermédiaire d'un monochromateur derrière l'une des fenêtres (4) - et un détecteur de rayonnement infrarouge (5) derrière l'autre fenêtre (4'), et comportant une unité de calcul (6) pour le traitement des valeurs de mesure de l'absorption (A), déterminées par celle-ci, et comportant une unité d'affichage pour afficher ou éditer au moins la teneur en éthanol ou un autre alcool dans le liquide (FI), le détecteur de rayonnement infrarouge (5) étant relié à l'unité de calcul (6) et à l'unité d'affichage.

13. Dispositif selon la revendication 12, **caractérisé en ce que** pour compenser ou corriger les variations de l'intensité du rayonnement émis par la source lumineuse infrarouge à diode électroluminescente (1), une unité de mesure de l'intensité du rayonnement, en particulier un photodétecteur, est associée à ladite source lumineuse ou est intégrée dans ou à celle-ci, laquelle unité de mesure est reliée par flux de données à l'unité de calcul (6).

2 – Durchflusszelle

100

1 NIR - LED mit  3 Wellenlängen

3 – Temperatur Sensor

5 - Detector

4, 4´ Fenster

Flüssigkeit fl
Flüssigkeitsprobe Pr

6

Fig. 1

EP 1 965 193 B1

Fig. 2

Fig. 3

Fig. 4a

Fig. 4b

Fig.4c

**EP 1 965 193 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5679955 A
- US 6690015 B1